(19) Europäisches Patentamt
European Patent Office
Office européen des brevets

(11) **EP 1 886 625 A1**

(12) **EUROPEAN PATENT APPLICATION**

(43) Date of publication:
**13.02.2008 Bulletin 2008/07**

(51) Int Cl.:
***A61B 5/0456*** (2006.01)

(21) Application number: **06254156.0**

(22) Date of filing: **08.08.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Jetfly Technology Limited Kowloon, Hong Kong (CN)**

(72) Inventor: **Wong, Kin Kai Andy Shatin New Territories Hong King (JP)**

(74) Representative: **Howe, Steven Marks & Clerk 90 Long Acre London WC2E 9RA (GB)**

(54) **QRS Estimation method and device**

(57) A method of estimating R-peaks in electrocardiographic (ECG) signals of an individual is disclosed as comprising the steps of (a) detecting ECG signals of the individual; (b) converting the ECG signals into digital data; and (c) determining the average gradients of successive groups of the digital data.

*Fig. 3*

EP 1 886 625 A1

## Description

This invention relates to a method and device for estimating QRS complexes in detected electrocardiographic (ECG) signals of an individual. Such a method and device are suitable for monitoring such physiological parameters as heart rate and blood pressure of the individual.

When a heart of an individual beats, electrical signals (called "ECG signals") flow through the heart, which may be picked up by appropriate devices attached to the skin of the individual. A typical and complete ECG signal includes a complete waveform, which is said to be comprised of a P wave, a Q wave, an R wave, an S wave and a T wave, in which the Q wave, R wave and S wave are sometimes collectively referred to as forming a "QRS complex". Generally speaking, the P-wave arises from the depolarisation of the atrium, the QRS complex arises from depolarisation of the ventricles, and the T wave arises from re-polarisation of the ventricle muscle. Generally, the R wave is the most prominent wave in the waveform of the whole ECG signal. The heart rate (or rate of heart beat) of an individual may thus be determined by detecting and measuring the time intervals between successive R waves of the ECG signals.

The challenge of QRS estimation arises from the fact that ECG signals and their noise level vary from person to person. Even for the same person, they vary from time to time. Furthermore, some people have weak ECG signals, i.e. the QRS peak is very small. For some people, they can have a high T wave, which may be easily mistakenly identified as the R wave.

Adaptive threshold, template matching and guarding period are the most commonly used techniques in many QRS detection algorithms to tackle these problems. However, each technique has its own shortcomings. Adaptive threshold method aims at adjusting the thresholds based on the incoming ECG signals. If the incoming signals are very noisy (especially motion artifacts) or there is any false detection, the thresholds will be wrong and such will affect the estimation thereafter. The recovering really depends on the design of the adaptive algorithm. In the worst case, it will not recover at all and will produce no QRS detection. Template matching method measures the similarity of the R-peak candidate relative to a reference template. However, obtaining a reference template that suits the majority of people is very difficult. Although an adaptive approach that uses the characteristic of the valid R-peak to generate the reference template can be used, the initial criteria for validating the first R-peak are still difficult to achieve. Any false detection will also affect the estimation thereafter. Guarding period technique rejects any R-peak candidate within a preset time window after a valid R-peak. It assumes the first R wave is correct. This approach helps in rejecting strong T-wave as well. However, the assumption that the first R wave is correct may not be justified and may cause false detection.

The aforementioned techniques may work well if the individual under measurement is closely monitored or the estimation is for post-processing. For portable ECG devices such as those in the form of a wrist-worn watch, with which the user is not monitored when using the device, motion artifacts could be the major factor affecting the results. The difficulty increases further when detection needs to be in real-time and with limited processing power. For example, if the device is a watch, the speed and memory of the MCU is limited. The number of samples that can be stored and processed will be small. These are not good operating conditions, in particular for many template-matching techniques.

According to a first aspect of the present invention, there is provided a method of estimating R-peaks in electrocardiographic (ECG) signals of an individual, comprising the steps of (a) detecting ECG signals of said individual; and (b) converting said ECG signals into a plurality of digital data; characterized in including a step of (c) determining the average gradients of successive groups of said digital data.

According to a second aspect of the present invention, there is provided a device for estimating R-peaks in electrocardiographic (ECG) signals of an individual, comprising means for detecting ECG signals of said individual; and means for converting said ECG signals into a plurality of digital data; characterized in including means for determining the average gradients of successive groups of said digital data.

An embodiment of the present invention will now be described with reference to the accompanying drawings, in which:

Fig. 1 is an exemplary electrocardiogram showing a series of ECG signals;
Fig. 2 is an exemplary electrocardiogram showing a series of ECG signal data as converted by an analog-to-digital (A/D) converter;
Fig. 3 is a flowchart showing the steps of a method of identifying R-peaks of ECG signals according to the present invention;
Fig. 4 is a flowchart showing the steps of "Feature Checking" in the flowchart of Fig. 3; and
Fig. 5 is a flowchart showing the steps of "R-Peak Verification" in the flowchart of Fig. 3.

To determine an R-peak candidate (i.e. a possible R-peak), a method and a device according to the present invention adopts an algorithm which examines four features of the ECG signals, namely (a) amplitude, (b) slope quality, (c) the existence of a peak, and (d) incident angle of the ECG signals.

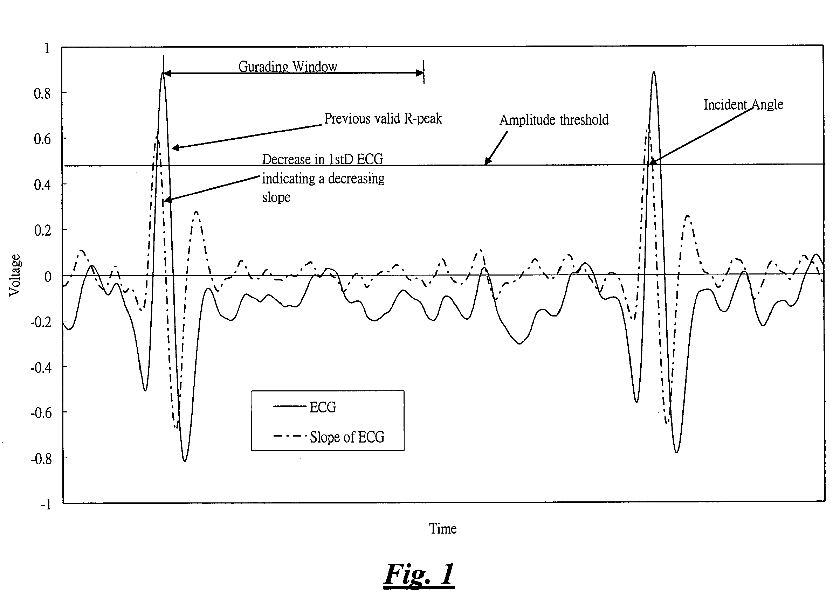

Fig. 1 is an exemplary electrocardiogram showing a series of ECG signals with two R-peaks. For the amplitude, the algorithm checks if the amplitude of the R-peak candidate is continuously larger than a pre-determined amplitude threshold value.

For slope quality, the algorithm will check if the R-peak candidate has generally continuously decreasing gradients, by determining the average gradients of successive groups of the ECG signals represented by converted digital data. Further details of this will be further discussed below.

For peak existence, the algorithm checks if a peak exists. The peak is defined as a sign change in gradient from positive to negative.

Finally, the incident angle is defined as the gradient/angle at which the R-peak candidate crosses the pre-determined amplitude threshold value. The algorithm will check if the incident angle is larger than a pre-determined threshold value. If checking of all the four features give positive results, an R-peak candidate is found. If the R-peak candidate is not within a guarding window of (i.e. not within a certain period of time from) the previous R-peak, it is confirmed as a valid R-peak. On the other hand, if the R-peak candidate has a larger incident angle than that of the previous R-peak, it will either replace the previous R-peak or is invalid, to be determined in a manner to be discussed below.

It should be noted that the amplitude threshold value, the incident angle threshold value and the size of the guarding window are not fixed, but are continuously updated based on incoming ECG signals. For the calculation of these threshold values, let us define the followings:

$\tilde{x}_M(n) = x_M(n) - \bar{x}(M-1)$ for n=0,1,...N-1 where

N = frame size

$$\tilde{x}_M(n) = \text{nth sample of frame M (ac value)} \quad \text{------------------------------------------}(1)$$
$$x_M(n) = \text{nth sample of frame M (ac + dc value)}$$

$$\bar{x}(M-1) = \frac{\sum_{n=0}^{N-1} x_{M-1}(n)}{N}$$

$$\tilde{x}_{max}(M) \text{ be the maximum value of } \tilde{x}_M(n) \text{ for } n = 0,1,...,N-1 \quad \text{-----------------------------}(2)$$

$$ECG\_th_M \text{ be the ECG amplitude threshold value for frame M} \quad \text{--------------------------} (3)$$

$$\angle x(n) = [\angle x_{(n)} - \angle x_{(n-10)}]/10 \text{ be the average gradient of the preceding 10 samples } (x_{(n-9)}, x_{(n-8)},$$
$$x_{(n-7)}, \ldots x_{(n)}) \text{ -------------------- (4)}$$

$$\angle x_{th} \text{ be the incident anlge threshold value} \quad \text{-------------------------------------------------------}(5)$$

The ECG amplitude threshold value, $ECG\_th_M$, is updated every two seconds in the following manner:

If time < 2 seconds then

$$ECG\_th_0 = 0.5\tilde{x}_{max}(0) + \bar{x}(0)$$

else if for every 2 seconds then
Update $\bar{x}(M)$

$$ECG\_th_{M+1} = 0.5\tilde{x}_{max}(M) + \bar{x}(M)$$

The incident angle threshold value, $\angle x_{th}$, is updated for every sample as shown below:

$\angle x_{th}$ is the 90% of the average of all $\angle x(n)$ that meets the criterion:

0 volt per second < $\angle x(n)$ < 100 volts per second

The bound check for the incident angle is to eliminate unreasonable incident angles caused by noise, especially motion artifacts.

The size of guarding window is based on the current heart rate (HR). The defualt values is 300ms. If there is a valid HR, the time window will be 0.5HR period. The starting of the window is the position where there is a valid R-peak.

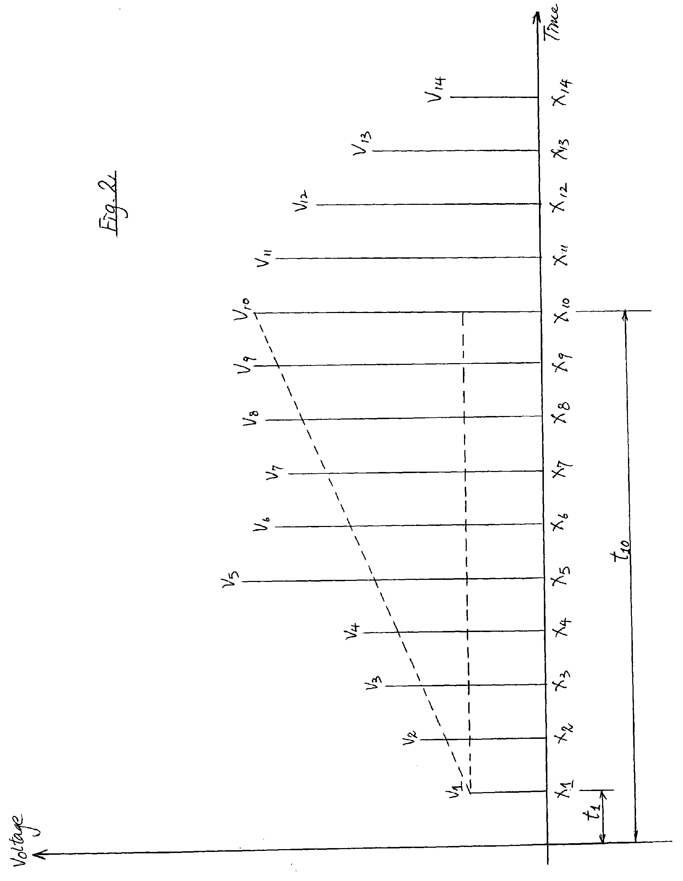

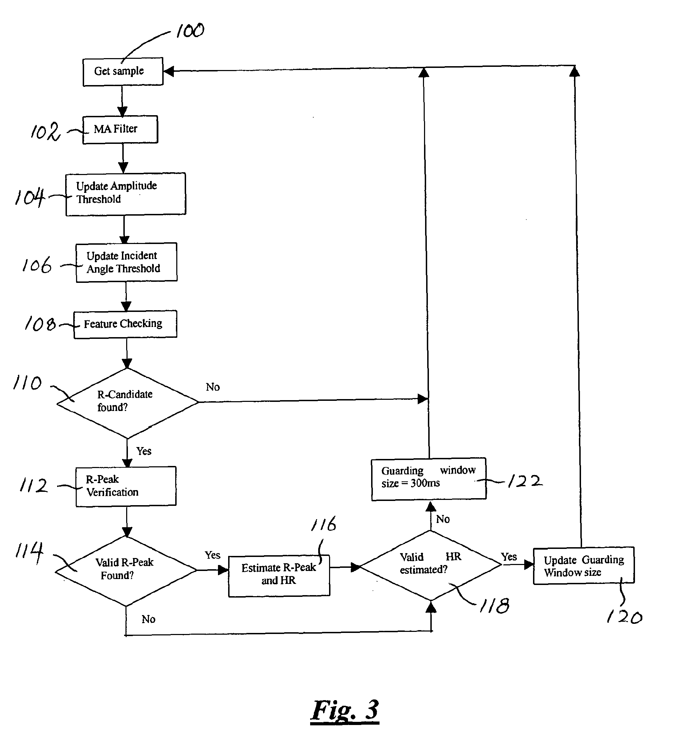

For real-time implemenation, the QRS estimation operates on a sample-by-sample basis. Figs. 3 to 5 show flow charts of the implementation. The sampling rate is 1kHz and, in paractice, all the gradients are average gradients over ten samples, in accordance with Equation 4 above. As shown in Fig. 2, and in accordance with Equation 4 above, the average gradient over samples $x_1$ to $x_{10}$ is:

$$\frac{V_{10} - V_1}{t_{10} - t_1}$$

As shown in Fig. 3, the input samples are used for updating the amplitude threshold value and incident angle threshold value before feature checking.

In particular, as shown in Fig. 3, samples are obtained (100) and passed through (102) a moving average (MA) filter, which acts as a low-pass filter. The incoming signals are converted by an analog-to-digital (A/D) converter into digital data for manipulation. The amplitude threshold is then updated (104) in accordance with the incoming ECG signals in the manner discussed above. Similarly, the incident angle threshold is also updated (106) in accordance with the incoming ECG signals in the manner discussed above. Feature checking (108) is then carried out. If no R-peak candidate is found (110), samples are continuously obtained (100). If, on the other hand, an R-peak candidate is found (110), R-peak verification process (112) will be carried out. If a valid R-peak is found (114), then an estimated heart rate (HR) will be calculated (116). The estimated HR value will then be checked (118). If the estiamted HR is within a preset range, e.g. between 30 beats per minute to 240 beats per minute, the size of the guarding window will be updated (120) in the manner discussed above. If not, the size of the guarding window will be set (122) as the default value, namely 300ms.

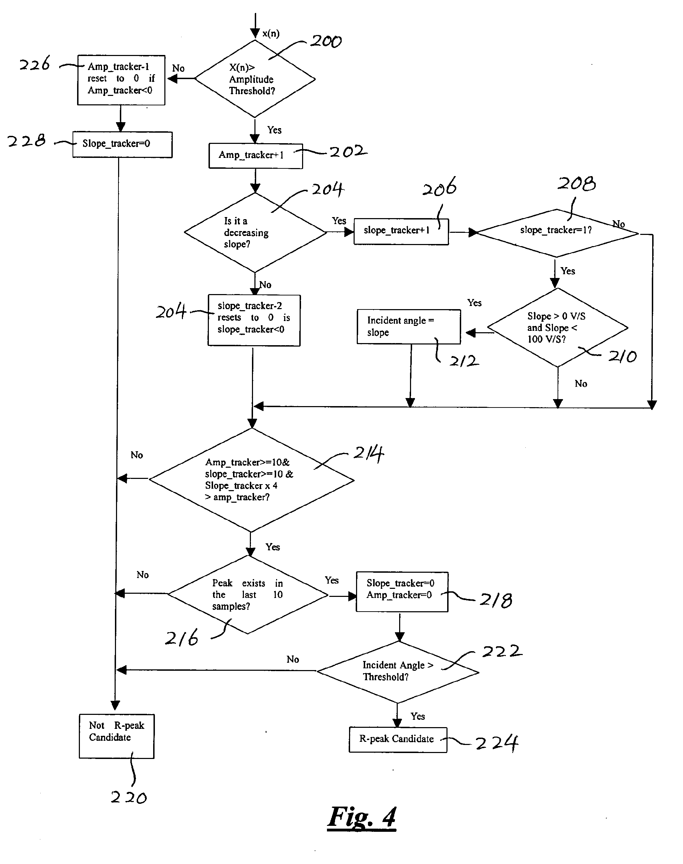

Fig. 4 is a flow chart showing in more detail the steps of feature checking (108). For the feature checking, two variables, namely *amp_fracker* and *slope_tracker,* are used for tracking the amplitude and slope quality of the incoming ECG signals, x(n), respectively.

The two trackers have different scoring schemes. Generally speaking, the *amp_tracker* will increase by 1 if the amplitude of the datum of the sample is larger than the amplitude threshold value, $ECG\_th_M$. Otherwise it decreases by 1, and if it is negative, it will be set to 0. For the *slope_tracker,* it will increase by 1 if it is of a decreasing gradient (i.e. of a gradient smaller than that of the immediately preceding datum or group of data). If, on the other hand, it is of an increasing gradient (i.e. of a gradient larger than or equal to that of the immediately preceding datum or group of data), it will decrease by 2. If the amplitude of the datum is not larger than $ECG\_th_M$ or if the *slope_tracker* is negative, the *slope_tracker* is set to 0.

The psuedo-code for the *amp_tracker* and *slope_tracker* scoring schemes is shown below:

$$\text{If } \widetilde{x}_M(n) > ECG\_th_M$$

$$\text{Amp\_tracker } +1;$$

$$\text{Else}$$

$$\text{Amp\_tracker } -1;$$

$$\text{Slope\_tracker } = 0;$$

$$\text{If Amp\_tracker } < 0$$

$$\text{Amp\_tacker } = 0;$$

$$\text{If } \angle x_M(n) < \angle x_M(n-1)$$

$$\text{Slope\_tracker } +1;$$

$$\text{Else}$$

$$\text{Slope\_tracker } -2;$$

$$\text{If Slope\_tracker } < 0$$

$$\text{Slope\_tracker } = 0;$$

….

….

$$\text{If peak found}$$

$$\text{Amp\_tracker } = 0;$$

$$\text{Slope\_tracker } = 0;$$

Since the *amp_tracker* and *slope_tracker* have different scoring schemes, it would be a reliable indication of an R-peak candidate if both variables show similar values. A condition of (*slope_tracker* x 4) > *amp_tracker* is used for ensuring that both trackers will not deviate too much. Once the *amp_tracker* and *slope_tracker* checking show positive results, the algorithm checks for peak existence within the last ten samples of the ECG. Once a peak is found, the trackers will be reset. The reset of trackers is important to prevent another peak checking in the next samples. Finally, it is the incident angle check and if the checking result is positive, an R-peak candidate is found. The inclination angle of the R-peak is the gradient at which the R-peak candidate starts to cross $ECG\_th_M$, which is when *slope_tracker* = 1.

Turning now to Fig. 4, upon receipt of sample x(n), it will be checked (200) whether the value of *x(n)* is larger than the updated amplitude threshold. If so, as stated above, the *amp_tracker* will increase by 1 (202). It will then be checked as to whether the gradient is less than that of the last sample (204). If so, and as stated above, the *slope_tracker* will increase by 1 (206). If the *slope_tracker* equals 1 (208), it will then check (210) whether the gradient is within a preset range, e.g. between 0 volt per second to 100 volts per second. If so, the incident angle will be the gradient of the sample *x(n)* (212). The algorithm will then check (214) (a) whether the *amp_tracker* is at least 10, (b) whether the *slope_tracker* is at least 10, and (c) whether (*slope_tracker* x 4) > *amp_tracker.* If any one of these conditions is not present, such will not be accepted as an R-peak candidate (220). If all these three conditions exist, it will check (216) whether a peak exists in the preceding ten samples. If not, then such will not be accepted as an R-peak candidate (220). If, on the other hand, a peak exists in the preceding ten samples, both the *slop_tracker* and the *amp_tracker* will be reset (218) as 0. The algorithm will then check whether the incident angle is larger than the threshold (222). If so, such will be accepted as an R-peak candidate (224).

If the amplitude of the sample *x(n)* is found to be less than the current amplitude threshold (200), the *amp_ tracker* will decrease by 1. If the current value of the tracker will then be negative, it will be set to 0 (226). The *slope_ tracker* will be set to be 0 (228). The sample *x(n)* is confirmed not to be an R-peak candidate (220). If the amplitude of the sample *x(n)* is found to be more than the current amplitude threshold (200), but the gradient of this sample is not less than that of the last sample (204), the *slope_tracker* will decrease by 2. If by decreasing by 2 the *slope_tracker* will become negative, the *slope_tracker* will be set as 0 (230). The algorithm will then check (214) (a) whether the *amp_ tracker* is at least 10, (b) whether the *slope_tracker* is at least 10, and (c) whether (*slope_tracker* x 4) > *amp_tracker,* and proceed with the remaining steps as discussed above. It can be seen that both the *slope_tracker* and *amp_tracker* are basically running sum algorithms, and two of the criteria to be checked for estimating an R-peak is whether both the running sums are at least 10.

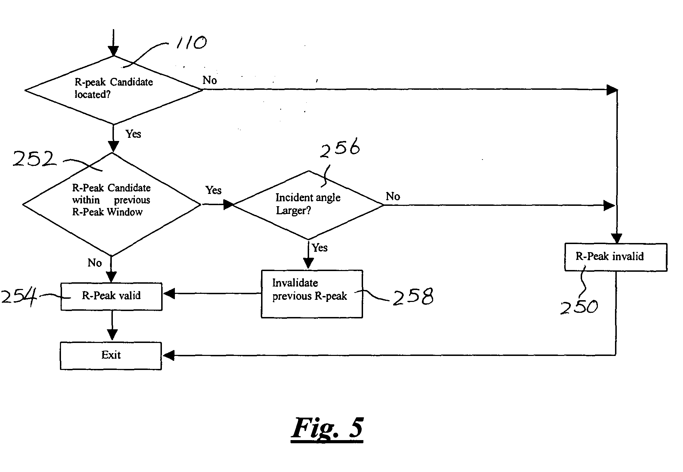

Fig. 5 is a flow chart showing the steps in the procedure of R-peak verification (112). The is to check if the R-peak candidate found in the previouis step (110) is within the gurading window of the previous valid R-peak. If this is the case and if the R-peak candidate has a larger incident angle than the previous valid R-peak, the R-peak candidate is confirmed to be valid and the previous R-peak will be invalidated. If the R-candidate is outside the guarding window, it is a valid R-peak and so is the previous R-peak. This verification procedure should filter out not only strong-T waves, but also most of the false R-peak due to noise. When a new R-peak is found, the peak location can be estimated.

More particularly, and referring to Fig. 5, if the incoming sample *x(n)* is not found to be an R-peak candidate (110), such will not be confirmed to be an R-peak (250), and further samples will then be obtained for processing (100). If, on the other hand, the sample *x(n)* is found to be an R-peak candidate (110), then it will check whether the occurrence of this R-peak candidate is within the guarding window of the previous R-peak (252). If not, then this R-peak candidate will be confirmed as a valid R-peak (252), and used for calculating, e.g. the heart rate of the one being monitored. If, on the other hand, the occurrence of this R-peak candidate is within the guarding window of the previous R-peak (252), then it will check (256) whether the incident angle of the R-peak candidate is larger than that of the previous confirmed R-peak. If not, this R-peak candidate will not be considered to be a valid R-peak (250). If, on the other hand, the incident angle of the R-peak candidate is larger than that of the previous confirmed R-peak (256), the previous R-peak will be invalidated (258), and the R-peak candidate will be confirmed as an R-peak (254).

It is found in practice that a device for monitoring heart rate and blood pressure adopting such a method requires little memory (e.g. as little as 128 bytes) and computation capability, which means that the hardware requirement, in particular in respect of the micro-controller unit, is reduced. Most of the memory is used for ECG signal buffers, which is only 20 memory locations. Such an algorithm is real-time and has a short response time. Although it does not make use of any template, it can identify QRS peaks with a wide range of subjects.

It should be understood that the above only illustrates an example whereby the present invention may be carried out, and that various modifications and/or alterations may be made thereto without departing from the spirit of the invention.

It should also be understood that various features of the invention which are, for brevity, described here in the context of a single embodiment, may be provided separately or in any appropriate sub-combinations.

**Claims**

1. A method of estimating R-peaks in electrocardiographic (ECG) signals of an individual, comprising the steps of:

   (a) detecting ECG signals of said individual; and
   (b) converting said ECG signals into a plurality of digital data;
   **characterized in** including a step of:
   (c) determining the average gradients of successive groups of said digital data.

2. A method according to Claim 1 further **characterized in** including a step (d) of checking for the existence of generally continuously decreasing gradients of said groups of said digital data.

3. A method according to Claim 1 further **characterized in** including a step (e) of checking whether the amplitude of said plurality of detected ECG signals exceed a pre-determined amplitude value.

4. A method according to Claim 3 further **characterized in that** said pre-determined amplitude value varies during the course of working of said method.

5. A method according to Claim 1 further **characterized in** including a step (f) of checking whether at least a first group of said data is of a positive gradient and at least a second subsequent group of data is of a negative gradient.

6. A method according to Claim 1 further **characterized in** including a step (g) of checking whether the gradient at which said first group of data whose amplitude is no less than said pre-determined amplitude value crosses said pre-determined amplitude value exceeds a pre-determined gradient value.

7. A method according to Claim 6 further **characterized in that** said pre-determined gradient value varies during the course of working of said method.

8. A method according to Claim 6 further **characterized in that** said pre-determined gradient value equals 90% of the average of all preceding incident angles, $\angle x(n)$, which meet the critera 0 volt per second $< \angle x(n) <$ 100 volts per second.

9. A method according to Claim 1 further **characterized in** further including a step (h) of checking whether an R-peak candidate occurrs within a pre-determined range of period of time after the immediately preceding R-peak.

10. A method according to Claim 9 further **characterized in** including a step (i) of comparing the incident angle of said R-peak candidate with the incident angle of the immediately preceding R-peak.

11. A method according to Claim 10 further **characterized in** including a step (1) of invalidating the immediately preceding R-peak if the incident angle of said R-peak candidate is larger than the incident angle of the immediately preceding R-peak.

12. A device for estimating R-peaks in electrocardiographic (ECG) signals of an individual, comprising:

   means for detecting ECG signals of said individual; and
   means for converting said ECG signals into a plurality of digital data;

   **characterized in** including:

   means for determining the average gradients of successive groups of said digital data.

13. A device according to Claim 12 further **characterized in** including means for checking for the existence of generally continuously decreasing gradients of said groups of said digital data.

14. A device according to Claim 12 further **characterized in** including means for checking whether the amplitude of said plurality of detected ECG signals exceed a pre-determined amplitude value.

15. A device according to Claim 14 further **characterized in that** said pre-determined amplitude value is adapted to vary during the course of working of said method.

16. A device according to Claim 12 further **characterized in** including means for checking whether at least a first group of said data is of a positive gradient and at least a second subsequent group of data is of a negative gradient.

17. A device according to Claim 12 further **characterized in** including means for checking whether the gradient at which said first group of data whose amplitude is no less than said pre-determined amplitude value crosses said pre-determined amplitude value exceeds a pre-determined gradient value.

18. A device according to Claim 17 further **characterized in that** said pre-determined gradient value is adapted to vary during the course of working of said method.

19. A device according to Claim 17 further **characterized in that** said pre-determined gradient value equals 90% of the average of all preceding incident angles, $\angle x(n)$, which meet the critera 0 volt per second $< \angle x(n) <$ 100 volts per second.

20. A device according to Claim 12 further **characterized in** including means for checking whether an R-peak candidate occurrs within a pre-determined range of period of time after the immediately preceding R-peak.

21. A device according to Claim 20 further **characterized in** including means for comparing the incident angle of said R-peak candidate with the incident angle of the immediately preceding R-peak.

**22.** A device according to Claim 21 further **characterized in** including means for invalidating the immediately preceding R-peak if the incident angle of said R-peak candidate is larger than the incident angle of the immediately preceding R-peak.

*Fig. 1*

EP 1 886 625 A1

EP 1 886 625 A1

Fig. 2

10

*100*

Get sample

*102* — MA Filter

*104* — Update Amplitude Threshold

*106* — Update Incident Angle Threshold

*108* — Feature Checking

*110* — R-Candidate found? — No →

Yes ↓

*112* — R-Peak Verification

*114* — Valid R-Peak Found? — Yes → Estimate R-Peak and HR *116* → Valid HR estimated? *118* — Yes → Update Guarding Window size *120*

No ↓ (from 118) Guarding window size = 300ms *122*

No (from 114)

*Fig. 3*

11

226 — Amp_tracker-1 reset to 0 if Amp_tracker<0  ←No— X(n)> Amplitude Threshold? — 200

x(n)

228 — Slope_tracker=0

Yes

Amp_tracker+1 — 202

204 — Is it a decreasing slope? —Yes→ slope_tracker+1 — 206 → slope_tracker=1? — 208 —No

No

Yes

204 — slope_tracker-2 resets to 0 is slope_tracker<0

Incident angle = slope — 212 ←Yes— Slope > 0 V/S and Slope < 100 V/S? — 210

No

No

214 — Amp_tracker>=10& slope_tracker>=10 & Slope_tracker x 4 > amp_tracker?

Yes

No

Peak exists in the last 10 samples? — 216 —Yes→ Slope_tracker=0 Amp_tracker=0 — 218

No

Not R-peak Candidate

Incident Angle > Threshold? — 222

220

Yes

R-peak Candidate — 224

## Fig. 4

110

R-peak Candidate located? — No →

Yes ↓

252

R-Peak Candidate within previous R-Peak Window — Yes → 256 Incident angle Larger? — No →

No ↓

Yes ↓

254 — R-Peak valid ← Invalidate previous R-peak — 258

R-Peak invalid

250

Exit ←

**_Fig. 5_**

**European Patent Office**

## EUROPEAN SEARCH REPORT

Application Number

EP 06 25 4156

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (IPC) |
| X | US 6 070 097 A (KREGER KEVIN S [US] ET AL) 30 May 2000 (2000-05-30) * column 4, line 30 - line 60 * * column 5, line 39 - column 69, line 67 * ----- | 1-22 | INV. A61B5/0456 |
| X | US 2004/186388 A1 (GERASIMOV VADIM [US]) 23 September 2004 (2004-09-23) <br><br> * paragraph [0017] * * paragraph [0020] - paragraph [0032]; claim 1 * ----- | 1-3, 6-14, 17-22 | |
| X | US 6 128 526 A (STADLER ROBERT [US] ET AL) 3 October 2000 (2000-10-03) * column 16, line 34 - line 65 * * column 18, line 46 - column 19, line 51; figure 8 * ----- | 1-3,9, 12-14,20 | |
| X | US 4 679 144 A (COX MICHAEL W [US] ET AL) 7 July 1987 (1987-07-07) <br><br> * column 6, line 25 - column 8, line 23 * ----- | 1-3,5,9, 12-14, 16,20 | |
| | | | TECHNICAL FIELDS SEARCHED (IPC) |
| X | US 2004/254611 A1 (PALREDDY SUREKHA [US] ET AL) 16 December 2004 (2004-12-16) <br><br> * paragraph [0031] - paragraph [0035] * * paragraph [0058] - paragraph [0061] * ----- | 1,2,5,9, 12,13, 16,20 | A61B G06F A61N |

The present search report has been drawn up for all claims

| Place of search | Date of completion of the search | Examiner |
|---|---|---|
| Berlin | 11 January 2007 | Trachterna, Morten |

EPO FORM 1503 03.82 (P04C01)

## ANNEX TO THE EUROPEAN SEARCH REPORT
## ON EUROPEAN PATENT APPLICATION NO.

EP 06 25 4156

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2007

| Patent document cited in search report | | Publication date | Patent family member(s) | | Publication date |
|---|---|---|---|---|---|
| US 6070097 | A | 30-05-2000 | NONE | | |
| US 2004186388 | A1 | 23-09-2004 | NONE | | |
| US 6128526 | A | 03-10-2000 | EP | 1164933 A1 | 02-01-2002 |
| | | | WO | 0057781 A1 | 05-10-2000 |
| US 4679144 | A | 07-07-1987 | AU | 4649785 A | 26-02-1987 |
| | | | CA | 1281081 C | 05-03-1991 |
| | | | DK | 373385 A | 22-02-1986 |
| | | | EP | 0176220 A2 | 02-04-1986 |
| | | | ES | 8706419 A1 | 16-09-1987 |
| | | | FI | 853046 A | 22-02-1986 |
| | | | JP | 61168333 A | 30-07-1986 |
| | | | NO | 853282 A | 24-02-1986 |
| US 2004254611 | A1 | 16-12-2004 | AU | 2004245030 A1 | 16-12-2004 |
| | | | CA | 2527558 A1 | 16-12-2004 |
| | | | EP | 1631350 A2 | 08-03-2006 |
| | | | JP | 2006526472 T | 24-11-2006 |
| | | | WO | 2004108212 A2 | 16-12-2004 |

EPO FORM P0459

For more details about this annex : see Official Journal of the European Patent Office, No. 12/82